(19) Europäisches Patentamt / European Patent Office / Office européen des brevets

(11) **EP 3 454 045 A1**

(12) **EUROPÄISCHE PATENTANMELDUNG**

(43) Veröffentlichungstag:
**13.03.2019 Patentblatt 2019/11**

(51) Int Cl.:
***G01N 21/3577*** *(2014.01)* ***G01N 33/28*** *(2006.01)*
***G01N 21/35*** *(2014.01)*

(21) Anmeldenummer: **17189615.2**

(22) Anmeldetag: **06.09.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Quantared Technologies GmbH**
**1100 Wien (AT)**

(72) Erfinder:
- **RITTER, Wolfgang**
  **1100 Wien (AT)**
- **LENDL, Bernhard**
  **1100 Wien (AT)**

(74) Vertreter: **Sonn & Partner Patentanwälte**
**Riemergasse 14**
**1010 Wien (AT)**

# (54) VERFAHREN ZUR QUANTITATIVEN BESTIMMUNG VON FETTSÄUREESTERN IN TREIBSTOFFEN

(57) Die vorliegende Erfindung betrifft ein Verfahren zur quantitativen Bestimmung von Fettsäureestern in Treibstoffen, wobei der Analyt einer für ihn selektiven, die Intensität der Carbonylbande der jeweiligen Estergruppe beeinflussenden chemischen Reaktion unter Bildung eines modifizierten Analyten unterzogen und die Änderung der Konzentration an Analyt in der Probe unter Heranziehung der Abnahme der Intensität der Carbonylbande und/oder die Zunahme der Konzentration des Bildung des modifizierten Analyten unter Heranziehung der Zunahme der Intensität einer für die Modifizierung charakteristischen Bande gemessen wird.

Fig. 3:

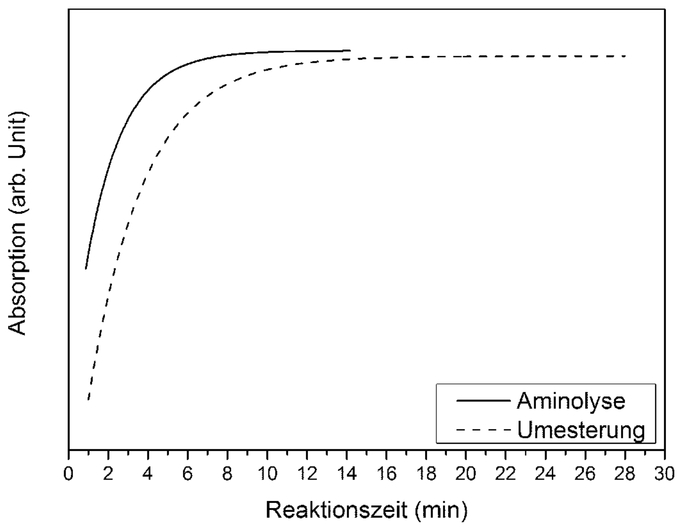


EP 3 454 045 A1

## Beschreibung

Die vorliegende Erfindung betrifft ein Verfahren zur quantitativen Bestimmung von Analyten basierend auf Ester, insbesondere Fettsäureester, wie z.B. Fettsäuremethyl- bzw. ethylester (FAME/FAEE) beziehungsweise Glyceriden in Treibstoffen wie z.B. Flugturbinentreibstoff (Jet Fuel).

Rasche und genaue Routinemessungen von Verunreinigungen, Additiven oder Bestandteilen von flüssigen Kraftstoffen zählen zu den Hauptaufgaben der industriellen Messtechnik. Kraftstoffe werden nach strengen Spezifikationen hergestellt, können aber während dem Transport in Pipelines, Tankern, Lagertanks oder Filtern verunreinigt werden. Zusätzlich ist es erlaubt Additive an verschiedenen Punkten der Lieferkette beizugeben.

Als Fettsäuremethylester bzw. fatty acid methyl ester (FAME) werden die Ester einer Fettsäure mit Methanol als Alkohol bezeichnet, entsprechend heißen die Ester einer Fettsäure mit Ethanol als Alkohol Fettsäureethylester bzw. fatty acid ethyl ester (FAEE). Gemische aus FAMEs und FAEEs, gewonnen aus pflanzlichen oder tierischen Fetten (z. B. Rapsöl), werden als Kraftstoff für Dieselmotoren genutzt und im Allgemeinen als Biodiesel bezeichnet. In vielen Ländern ist die Beimischung von Biodiesel zu Dieselkraftstoff bereits gesetzlich vorgeschrieben. Zusätzlich zu FAME und FAEE können auch Spuren deren Ausgangsprodukte (Glyceride) im Kraftstoff enthalten sein.

FAME, FAEE und Glyceride sind bei Raumtemperatur fest oder flüssig und kommen mit einem Teil ihrer Eigenschaften denen von Dieselkraftstoff sehr nahe, sind jedoch zugleich Lösungsmittel mit anderen Wirkungen als Dieselkraftstoff, was zu technischen Problemen an Dichtungsmaterialien in Motorensystemen führen kann. Fettsäuremethylester und -ethylester können daher grundsätzlich nur dann als alternativer Kraftstoff verwendet werden, wenn die Dichtungsmaterialien im verwendeten Motor gegen FAME bzw. FAEE beständig sind.

In den heutzutage verwendeten weltweiten Versorgungssystemen, wie Pipelines, Tanklastwägen, Zügen und Schiffen, werden oft die verschiedensten Treibstoffe und Flüssigkeiten nacheinander transportiert, diese jedoch unter Verwendung von Schutzmaßnahmen, die dafür ausgelegt sind um Kreuz-Kontaminierung zu minimieren. Speziell FAME hat jedoch die Eigenschaft, fest an Oberflächen zu haften, wodurch kleine Mengen an FAME auch in Düsentreibstoffen (Jet Fuel) gefunden werden können. Diese Verunreinigungen rührt zumeist davon, dass Jet Fuel nach FAME und/oder FAEE enthaltenden Treibstoffen im gleichen Transportsystem geliefert wurden. Zusätzlich können FAME und FAEE aus Biodiesel in Multiprodukt-Pipelines an den Wänden haften bleiben und somit nachfolgendes Jet Fuel verunreinigen.

Von der Joint Inspection Group (JIG) wurden FAME in Jet Fuel als gefährliche Substanzen eingestuft. Studien zeigen bereits bei geringer Konzentration potentielle Gefahren. Diese reichen von der Korrosion von Lagern, Ablagerungen in der Treibstoffdüse, Beeinträchtigungen von Dichtmaterialen bis zu Veränderung der Viskosität des Treibstoffes bei geringen Temperaturen in großer Höhe. Die internationalen Spezifikationen Def.Stan. 91.-91 und ASTM D1655 schreiben zurzeit ein Limit von < 50 mg/kg FAME in Jet Fuel vor. Bei höheren Werten wird ein Betanken der Flugzeuge und Starten der Triebwerke verboten bzw. sind bei irrtümlichem Betrieb von Triebwerken mit Jet Fuel mit mehr als 50 mg/kg FAME zwingend zusätzliche Wartungsarbeiten für die Triebwerke vorgeschrieben.

Zur Bestimmung des FAME/FAEE Gehalts in Jet Fuel werden zum gegenwärtigen Zeitpunkt im Wesentlichen vier Verfahren eingesetzt. Dabei handelt es sich um drei chromatographische Verfahren, die Gaschromatographie-Massenspektroskopie (GC-MS), die Hochleistungsflüssigkeitschromatographie (HPLC) und die Heartcut-Gaschromatographie; sowie ein Fourier-Transformations-Infrarot-Spektrometrie (FTIR) Verfahren. Zur quantitativen Konzentrationsbestimmung von FAME/FAEE/Glyceride wird zurzeit vor allem GC-MS eingesetzt. Dieses Verfahren ist zwar genau und spezifisch, kann jedoch nur in einem qualifizierten Analysenlabor durchgeführt werden und hat für die Analyse einen hohen Zeitbedarf. Zusätzlich können mit chromatographischen Verfahren kurzkettige FAME, z.B. Kokosnuss Methyl Ester, nicht von anderen Bestandteilen des Jet Fuels unterschieden werden.

Es besteht also der dringende Bedarf an einem vereinfachten Analyseverfahren für FAME, um idealerweise Vor-Ort- oder in einem Feldlabor Analysen durchführen zu können.

In den letzten Jahren wurde ein FTIR Analysenverfahren entwickelt, bei welchem die zu analysieren Probe (Jet Fuel + FAME) durch eine Einweg-Kartusche geleitet wird, welche mit einen Sorbensmaterial (beispielsweise Silikagel) gefüllt ist. Durch dieses Sorbensmaterial werden FAME mittels Adsorption innerhalb der Kartusche festgehalten, wodurch die hindurchgeleitete Probe danach frei von FAME ist. Mittels einer FTIR-Messung wird die Probe vor und nach der Kartusche verglichen, um damit den FAME-Gehalt zu bestimmen. Dafür wird vor allem die Bande der Carbonylgruppe um 1749 $cm^{-1}$ ausgewertet. Nachteilig daran ist, dass das Sorbensmaterial zumeist eine nicht ausreichende Selektivität für FAME zeigt, sodass auch andere (polare) Substanzen adsorbiert werden. Daher ist bei diesem Analyseverfahren zusätzlich ein chemometrisches Modell (PLS) notwendig, um den Einfluss dieser Störsubstanzen herauszurechnen.

Aufgabe der vorliegenden Erfindung ist es daher, ein Messverfahren für Ester, insbesondere Fettsäureester (FAME/FAEE aber auch Glyceriden) in Treibstoffen wie z.B. Düsentreibstoffen (Jet Fuel) zur Verfügung zu stellen, welches Messverfahren die Nachteile des Standes der Technik nicht aufweist. Dazu wird in einem Verfahren zur quantitativen Bestimmung von Fettsäureester, wie Fettsäuremethyl- bzw. etylester und/oder Glyceriden, in Treibstoffen der Analyt (z.B. FAME/FAEE/Glyceride) einer für ihn selektiven, die Intensität der Carbonylbande der jeweiligen Estergruppe beeinflussenden chemischen Reaktion unter Bildung eines modifizierten Analyten unterzogen und die Änderung der

Konzentration an Analyt in der Probe unter Heranziehung der Abnahme der Intensität der Carbonylbande und/oder die Zunahme der Konzentration des modifizierten Analyten unter Heranziehung der Zunahme der Intensität einer für die Modifizierung charakteristischen Bande gemessen. Durch die gegenüber der im Stand der Technik verwendeten Adsorption höhere Selektivität der selektiven chemischen Reaktion auf den Analyten kann eine höhere Selektivität des Messverfahrens erreicht werden. Durch den Vergleich mit der ursprünglichen, nicht reagierten Probe (enthaltend den Analyten) als spezifischen Hintergrund lassen sich auch Matrix-Effekte korrigieren. Deutlich weniger Störstoffe gehen diese selektive chemische Reaktion ein und damit wird das Verfahren für verschiedenste Proben robuster und weniger störempfindlich. Die Nachweisgrenze kann gesenkt werden, die höhere Selektivität der "Probenvorbereitung" erlaubt es, mit einzelnen Wellenlängenpunkten im Spektrum eine robuste Auswertung zu erzielen. Damit gelingt es in einer bevorzugten Ausführungsform des erfindungsgemäßen Verfahrens, ein spektral weit durchstimmbares FTIR durch Lichtquellen, wie zum Beispiel Quantenkaskadenlaser (QCL), mit limitierter spektraler Durchstimmbarkeit zu ersetzen. Vorteilhaft an dem erfindungsgemäßen Verfahren sind:

- höhere Selektivität
- höhere Robustheit gegenüber Störstoffen in verschiedenen Jet Fuel
- verbesserte Nachweisstärke
- Miniaturisierung durch QCL
- reduzierte Herstellkosten

Gemäß einer bevorzugten Ausführungsform der vorliegenden Erfindung erfolgt die Messung der Änderung der Konzentration an Analyt durch IR-Spektroskopie.

Günstig ist dabei, wenn die Messung der Änderung der Konzentration an Analyt durch FTIR-Spektroskopie erfolgt.

Auch hat sich als günstig herausgestellt, wenn die Messung der Änderung der Konzentration an Analyt durch IR-Spektroskopie unter Verwendung von Quantenkaskadenlaser erfolgt. Insbesondere durch Verwendung der QCL ist es möglich, die Änderung der Intensität einer Bande bei Analyten oder modifizierten Analyten in einem kleinen spektralen Fenster auswerten zu können

Jet Fuel ist kein Reinstoff, sondern ein Gemisch aus Paraffinen, Naphthenen und Aromaten in verschiedenen Verhältnissen. Durch die Zugabe von Additiven wird je nach Zusammensetzung garantiert, dass das Gemisch die notwendigen Spezifikationen erfüllt. Daraus folgt, dass Jet Fuel aus verschiedenen Raffinerien, Pipelines oder sogar Batches aus derselben Quelle spektral gesehen sehr unterschiedlich sein kann. Deshalb ist es im Allgemeinen mit optischen Messmethoden nicht möglich, geringe Konzentrationen von Bestandteilen in Jet Fuel ohne Generation eines probenspezifischen Hintergrunds zu messen.

Beim erfindungsgemäßen Verfahren wird die Probe (Jet Fuel + FAME/FAEE/Glycerid) im Zuge der Messung einer selektiven Reaktion unterzogen, so wird beispielsweise gemäß einer bevorzugten Ausführungsform der vorliegenden Erfindung FAME/FAEE/Glycerid oder allgemein ein zu bestimmender Ester bzw. Fettsäureester durch Zugabe eines Amins in das entsprechende Amid umgewandelt, wobei die Änderung der Konzentration an FAME/FAEE/Glycerid bzw. allgemein die Änderung der Konzentration an zu bestimmenden Ester bzw. Fettsäureester in der Probe anhand der Abnahme der Intensität der Carbonylbande des Esters und/oder der Zunahme der Intensität der Amidbande des gebildeten Amids gemessen werden kann.

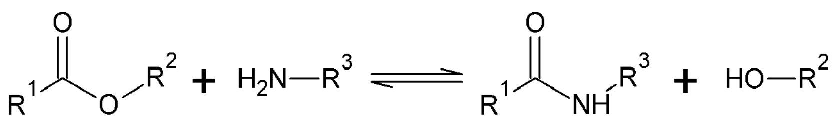

*Aminolyse eines Esters.*

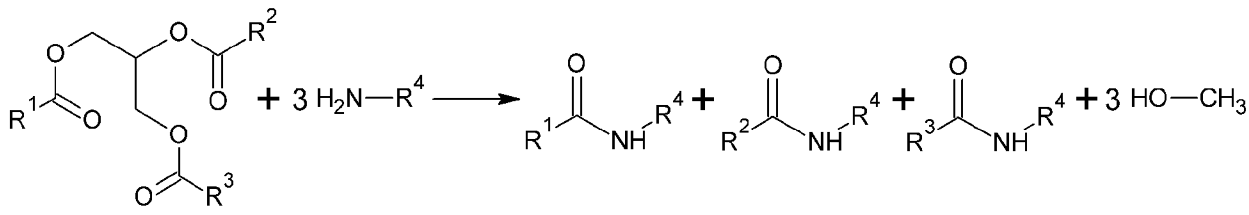

Aminolyse eines Triglycerids.

Gemäß einer weiteren bevorzugten Ausführungsform des erfindungsgemäßen Verfahrens wird der Ester bzw. FAME/FAEE/Glycerid durch Zugabe eines anderen Alkohols unter geeigneten Reaktionsbedingungen in den entsprechenden anderen Ester umgewandelt, wobei die Änderung der Konzentration an FAME/FAEE/Glycerid bzw. allgemein die Änderung der Konzentration an zu bestimmenden Ester bzw. Fettsäureester in der Probe anhand der Abnahme der Intensität der Carbonylbande des Esters und/oder der Zunahme der Intensität der Carbonylbande des anderen Esters gemessen wird. Die selektive Reaktion ist dabei eine Umesterung der Fettsäureester durch Zugabe eines anderen Alkohols. Der gebildete Ester (d. h. der modifizierte Analyt) zeigt eine verschobene Bande der Carbonylgruppe, welche von der ursprünglichen Bande unterschieden werden kann. Ausgewertet wird wiederum die Abnahme der Carbonylbande des Analyts Ester, FAME/FAEE bzw. des Glycerids und/oder die Zunahme der Carbonylbande des modifizierten Analyts. Leicht nachteilig kann bei diesem Verfahren der Umesterung eine spektrale Nähe der Produktbande zur Messwellenlänge und damit eine mögliche Beeinflussung der Messung sein.

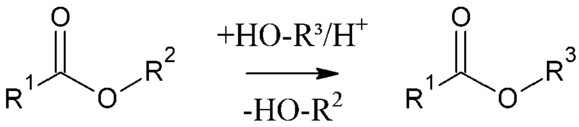


Umesterung mittels eines Alkohols.

In beiden dargestellten Reaktionen ändern sich die spektralen Eigenschaften der Probe, wodurch eine Bestimmung des Gehalts des Analyten mittels Vergleichsmessungen möglich wird. Dabei kann sowohl die Abnahme der Intensität der Carbonylbande des Analyten als auch die Zunahme der Intensität einer spezifischen Bande im modifizierten Analyten für das erfindungsgemäße Messverfahren herangezogen werden, wobei bei Berücksichtigung beider Änderungen sowie der Beziehung der Änderungen zueinander die quantitative Bestimmung des Analyten weiter vereinfacht wird.

Die beiliegende Fig. 1 zeigt den Abbau von FAME (1) anhand der Abnahme der Carbonylstreckschwingung um 1749 $cm^{-1}$ und die spektral hinreichend verschobene Zunahme des entsprechenden Amids als Reaktionsprodukt (2) im Infrarot-Absorptionsspektrum.

Gemäß einer bevorzugten Ausführungsform der vorliegenden Erfindung erfolgt die Reaktion zur Bildung des modifizierten Analyten enzymatisch katalysiert, besonders bevorzugt in Anwesenheit von Lipase. Auch die Verwendung anderer Enzyme ist denkbar, wobei diese Enzyme, bzw. Katalysatoren im Allgemeinen, die Reaktion zur Modifizierung des Analyten möglichst vollständig ablaufen lassen sollen.

Durch die selektive chemische Reaktion wird die Konzentration an FAME/FAEE/Glyceriden in der Probe verringert, dadurch kommt es im Fall von FAME im Infrarot-Spektrum zu einer Abnahme/Verschiebung der Carbonylbande (1749 $cm^{-1}$) bzw. im Fall der Aminolyse zu einem Hinzutreten einer Amid-Bande (1690 $cm^{-1}$). Die Abnahme der ursprünglichen Carbonylbande wird vermessen und über eine Kalibration in die gesuchte Analyt-Konzentration umgerechnet. Eine weitere mögliche Ausführungsform ist die Bestimmung der Konzentration des Analyten über die Messung der Zunahme der Intensität der Amidbande.

Die Carbonylgruppe der FAEE zeigt ihr Absorptionsmaxima bei 1742 $cm^{-1}$, gemäß einer bevorzugten Ausführungsform der vorliegenden Erfindung kann beispielsweise durch symmetrische Auswertung um 1742 $cm^{-1}$ die Konzentration an FAEE bestimmt bzw. herausgerechnet werden. Unter symmetrischer Auswertung wird dabei insbesondere eine Auswertung bei +/- 2 $cm^{-1}$, insbesondere +/- 5 $cm^{-1}$, besonders bevorzugt +/- 10 $cm^{-1}$ um die genannten Absorptionsmaxima verstanden, selbstverständlich kann die Bestimmung der Konzentration an Analyten aber auch gemäß anderen, dem Fachmann bekannten Verfahren erfolgen.

Eine mögliche unerwünschte Nebenreaktion bei Verwendung der Aminolyse im erfindungsgemäßen Verfahren ist die Umwandlung von FAME/FAEE/Glyceride mittels Alkoholen in andere Ester. Diese Ester zeigen ihr Absorptionsmaxima bei ca. 1742 $cm^{-1}$, welches eine Messung bei 1749 $cm^{-1}$ stören kann. Durch die oben erwähnte symmetrische

Auswertung um 1742 $cm^{-1}$ kann dieser Effekt ebenfalls herausgerechnet bzw. minimiert werden.

In den beiliegenden Figuren zeigen:

Fig. 1: Abbau von FAME (1) anhand der Abnahme der CarbonylStreckschwingung um 1749 cm-1 und der spektral hinreichend verschobenen Zunahme des entsprechenden Amids als Reaktionsprodukt (2) im Infrarot Absorptionsspektrum.

Fig. 2: Kinetik der enzymatisch-katalytischen Reaktion von FAME und Amin zum entsprechenden Amid (Punkte) mit exponentielle Fit (Linie) zur Messwertvorhersage.

Fig. 3: Vergleich der Reaktionskinetik der Umesterung und der Aminolyse.

Die vorliegende Erfindung wird im Folgenden anhand der genannten Beispiele erläutert, wobei sich die Erfindung nicht auf die Beispiele beschränkt.

Folgende Bestimmungen wurden durchgeführt:

- FAME in Jet Fuel mittels Aminolyse
- FAEE in Jet Fuel mittels Aminolyse
- Glyceryl trioleate in Jet Fuel mittels Aminolyse
- FAME in Diesel mittels Aminolyse
- FAME in Jet Fuel durch Umesterung

Für die Bestimmung von FAME in Jet Fuel mittels Aminolyse wurden zusätzliche Messungen vorgenommen:

- zugelassene Jet Fuel Additive
- Jet Fuel Screening.

Allgemeiner Methodenablauf

Messgeräte zur Durchführung der Methode bestehen in einer bevorzugten Ausführung aus einer Flusszelle, einer Infrarotquelle (z.B.: Globar mit oder ohne spektralem Filter oder QCL) samt Detektor, Pumpen, Ventilen, einem System zur Zudosierung des Reaktanten sowie einer Mehrweg-Kartusche die den Katalysator enthält. Die Schichtdicke der Flusszelle wird typischerweise auf die Lichtleistung der verwendeten Infrarotquelle und die IR Transmission der Probenmatrix abgestimmt.

Am Beginn einer Messsequenz wird das Messgerät mit der zur analysierenden Probenflüssigkeit gespült, danach wird ein Hintergrund mit QCL oder FTIR aufgenommen. Nach Zugabe eines der nachstehend angeführten Reaktanden wird die Probenflüssigkeit zwischen Flusszelle und einer den Katalysator enthaltenden Kartusche im Kreis gepumpt. Dabei wird der Analyt einer für ihn selektiven, die Intensität der Carbonylbande der jeweiligen Estergruppe beeinflussenden chemischen Reaktion unter Bildung eines modifizierten Analyten unterzogen und die Änderung der Konzentration an Analyt in der Probe unter Heranziehung der Abnahme der Intensität der Carbonylbande und/oder die Zunahme der Konzentration des Bildung des modifizierten Analyten unter Heranziehung der Zunahme der Intensität einer für die Modifizierung charakteristischen Bande gemessen. Über die Kinetik der Reaktion lassen sich frühe Schwellwertwarnungen abgegeben, der vollständige Ablauf der Reaktion überprüfen, sowie durch Vermessung von Standardproben die verbleibende Aktivität des Katalysators in der Mehrweg-Kartusche bestimmen. Nach Ablauf der Reaktion wird das Messgerät wieder mit Probenflüssigkeit gespült um dann einen zweiten Hintergrund zu messen. Aus der Änderung der gemessenen Intensität zwischen Hintergrund ($I_0$) und abreagierter Probe ($I$) wird mittels dem Lambert-Beer'schen Gesetz

$$Abs = \log (I_0/I)$$

die Absorption (Abs) bestimmt. Daraus ergibt sich, im Vergleich mit Kalibrationskurven, über

$$\text{Konzentration Analyt [mg/kg]} = (Abs * k + d) * \rho_C/\rho_S$$

die Konzentration des Analyten, wobei

*k* = Steigung der Kalibrationsgerade
*d* = Achsenabschnitt der Kalibrationsgerade

$\rho_C$ = Dichte der Kalibrationsmatrix in $kg/m^3$
$\rho_S$ = Dichte der Probe in $kg/m^3$

gilt.

Als Beispiel ergibt sich für eine Messung von Jet Fuel, das mit 49,9 mg/kg FAME versetzt wurde, bei einer gemessenen Absorption von 17,81 mAU mit der Kalibrationsgeraden k = 2,79 mAU-1 und d = -0,18 mg/kg eine berechnete FAME Konzentration von 49,51 mg/kg. Die Dichte der Kalibrationsmatrix und der Probe sind in diesem Beispiel identisch.

FAME in Jet Fuel mittels Aminolyse:

Die Aminolyse hat sich als vorteilhafte Variante herausgestellt, da sie unter enzymatisch-katalytischen Bedingungen für FAME Konzentrationen unter 500 mg/kg in unter 20 Minuten abgeschlossen ist (siehe beiliegende Fig. 2).

*Tabelle 1: FAME in Jet Fuel mittels Aminolyse.*

| Matrix | Analyt | Beigemengte Konzentration [mg/kg] | Berechnete Konzentration [mg/kg] |
|---|---|---|---|
| Jet Fuel | FAME Mix aus B7 | 0,0 | -0,02 |
| Jet Fuel | FAME Mix aus B7 | 5,5 | 5,34 |
| Jet Fuel | FAME Mix aus B7 | 11,1 | 11,26 |
| Jet Fuel | FAME Mix aus B7 | 22,1 | 21,66 |
| Jet Fuel | FAME Mix aus B7 | 199,3 | 200,19 |
| Jet Fuel | FAME Mix aus B7 | 400,4 | 399,98 |

Tabelle 1 zeigt exemplarische Resultate von Jet Fuels, die mit einem FAME-Mix (20 % RME Rape-Methyl-Ester, PME Palm-Methyl-Ester, TME Tallow-Methyl-Ester, SME Soy-Methyl-Ester und UCOME Used-Cooking-Oil-Methyl-Ester als siebenprozentige Lösung in Diesel; B7) in verschiedenen Konzentrationen versetzt wurden.

Die Resultate zeigen, dass FAME in Jet Fuel im Bereich 0 - 250 mg/kg mit einer Genauigkeit von < 1 mg/kg gemessen werden kann. Zur Bestimmung von FAME in Jet Fuel ohne den Einfluss von FAEE und Alkoholen wurden alle Daten symmetrisch um 1742 $cm^{-1}$ ausgewertet.

Das erfindungsgemäße Verfahren wurde nicht nur mit dem zuvor genannten, auf den europäischen Markt abgestimmten FAME Mix getestet, sondern auch mit einzelnen FAME Typen. Die Messergebnisse mit Konzentration um das erlaubte Maximum von 50 mg/kg sind in der untenstehenden Tabelle 2 aufgelistet.

Aus Tab. 2 wird ersichtlich, dass die Genauigkeit auf einzelne FAME etwas geringer ist, da sich FAME aus verschiedenen Quellen typischerweise in der Kettenlänge unterscheiden. Bei Short Chain Methyl Estern wie CME (Coconut-Methyl-Ester, wird vor allem in Südostasien verwendet und ist deshalb nicht Teil des auf Europa ausgelegten FAME Mixes) mit kürzeren Kohlenstoffkettenlängen und damit geringerem Molgewicht führt die Beigabe einer Konzentration in mg/kg zu einer größeren Menge an Carbonylgruppen und damit zu einer bekannten systematischen Überbestimmung. Diese Abweichungen lassen sich durch eine gezielte Kalibration leicht und einfach korrigieren. Die zurzeit zugelassenen Methoden können CME entweder nicht bestimmen (GC-MS, GC-Heart Cut und HPCL), oder haben dieselbe systematische Überbestimmung (FTIR Rapid Screening Method).

*Tabelle 2: FAME einzeln im Vergleich zur FAME Mix aus B7 Kalibration.*

| Matrix | Analyt | Beigemengte Konzentration [mg/kg] | Berechnete Konzentration [mg/kg] |
|---|---|---|---|
| Jet Fuel | RME aus B7 | 48,5 | 54,77 |
| Jet Fuel | PME aus B7 | 48,4 | 49, 44 |
| Jet Fuel | TME aus B7 | 47,7 | 48,48 |
| Jet Fuel | SME aus B7 | 49, 0 | 46,86 |
| Jet Fuel | UCOME aus B7 | 48,7 | 47,37 |
| Jet Fuel | CME aus B7 | 48,1 | 66,50 |

***Additive:***

Bei der maximalen in Jet Fuel erlaubten Konzentration von 15 zugelassenen Additiven verschiedener Typen (Static Dissipator, Lubricity Improver, Fuel System Icing Inhibitor, Metal Deactivator, Antioxidant & Cetane Improver, Biocide) wurde eine Messwertabweichung von < +/- 5 mg/kg gemessen. Dies liegt im Rahmen der erwarteten Methodenreproduzierbarkeit.

*Jet Fuel* ***Screening:***

Die Bestimmung von FAME in 26 unterschiedlichen Jet Fuels zeigte, dass durchaus Abweichungen vom Nominalwert auftreten können (73 % ≤ 5 mg/kg, bzw. 92 % ≤ 7,5 mg/kg). Solche und gravierendere Abweichungen sind aus weltweiten Jet Fuel Erhebungen des britischen Energy Institutes bekannt (n=189; 85% < 5 mg/kg, bzw. 95% < 10 mg/kg, gemessen mit FTIR Rapid Screening Method) und lassen sich auf die unterschiedlichen Zusammensetzungen und damit die spektralen Unterschiede von realen Jet Fuels zurückführen.

FAEE in Jet Fuel mittels Aminolyse

Die Aminolyse funktioniert ebenfalls mit Fatty Acid Ethyl Ester (FAEE), wobei jedoch im Vergleich zu FAME an geänderten Wellenzahlen ausgewertet werden muss. Tabelle 3 zeigt die Resultate einer Kalibration mit FAEE, in diesem Fall Ethylpalmitat, in verschiedenen Konzentrationen bei einer symmetrischen Auswertung um 1749 $cm^{-1}$.

*Tabelle 3: FAEE in Jet Fuel mittels Aminolyse.*

| Matrix | Analyt | Beigemengte Konzentration [mg/kg] | Berechnete Konzentration [mg/kg] |
|---|---|---|---|
| Jet Fuel | FAEE | 0 | 0,89 |
| Jet Fuel | FAEE | 50 | 48,66 |
| Jet Fuel | FAEE | 100 | 100,23 |
| Jet Fuel | FAEE | 200 | 200,22 |

In Proben, welche sowohl FAEE als auch FAME enthalten, lassen sich durch die Wahl der Auswertewellenzahlen die beiden Analyten unabhängig voneinander bestimmen.

Tabelle 4 zeigt beispielhaft die Bestimmung von FAME aus einer Probe in der sowohl FAME als auch FAEE enthalten ist.

*Tabelle 4: Bestimmung von FAME in einer FAME & FAEE Mischung.*

| Matrix | Analyt | Beigemengte Konzentration FAME [mg/kg] | Beigemengte Konzentration FAEE [mg/kg] | Berechnete Konzentration FAME [mg/kg] |
|---|---|---|---|---|
| Jet Fuel | FAME & FAEE | 0 | 103,5 | -3,29 |
| Jet Fuel | FAME & FAEE | 198, 4 | 103,5 | 196,26 |

Glyceryltrioleat in Jet Fuel mittels Aminolyse

Die Aminolyse von Triglyceriden führt zur Bildung von Amiden und Alkohol. Diese Reaktion wurde mit Glyceryltrioleat in Jet Fuel mit Lipase als Katalysator durchgeführt. Tabelle 5 zeigt die Resultate für verschiedene Konzentrationen.

*Tabelle 5: Triglyceride in Jet Fuel mittels Aminolyse.*

| Matrix | Analyt | Beigemengte Konzentration [mg/kg] | Berechnete Konzentration [mg/kg] |
|---|---|---|---|
| Jet Fuel | Glyceryltrioleat | 0,0 | -0,98 |
| Jet Fuel | Glyceryltrioleat | 5,0 | 3,14 |
| Jet Fuel | Glyceryltrioleat | 19,9 | 20,18 |
| Jet Fuel | Glyceryltrioleat | 49,8 | 48,65 |
| Jet Fuel | Glyceryltrioleat | 98,5 | 99,61 |

FAME in Diesel mittels Aminolyse

Um zu zeigen, dass das erfindungsgemäße Verfahren auch auf andere flüssige Kraftstoffe ausgeweitet werden kann, wurden Spuren von FAME in einer Diesel B0 Matrix vermessen. Die Resultate der enzymatisch-katalytischen Aminolyse von FAME in Diesel sind in Tabelle 6 aufgelistet.

*Tabelle 6: FAME in Diesel mittels Aminolyse.*

| Matrix | Analyt | Beigemengte Konzentration [mg/kg] | Berechnete Konzentration [mg/kg] |
|---|---|---|---|
| Diesel | FAME Mix aus B7 | 0,0 | -0,27 |
| Diesel | FAME Mix aus B7 | 10,0 | 10,27 |
| Diesel | FAME Mix aus B7 | 19,9 | 20,86 |
| Diesel | FAME Mix aus B7 | 49,2 | 50,19 |
| Diesel | FAME Mix aus B7 | 198,7 | 198,08 |

FAME in Jet Fuel durch Umesterung

Die Umesterung von FAME mit Alkoholen führt zur Bildung von anderen Estern. Durch den geringeren Abstand der charakteristischen Banden zwischen Analyt und Reaktionsprodukt im Vergleich zur Aminolyse, ergeben sich bei der Umesterung etwas schlechtere Werte für Methodenreproduzierbarkeit und Messwertabweichung. Aus der in Fig. 3 gezeigten Reaktionskinetik wird ersichtlich, dass die Umesterung ca. zehn Minuten länger dauert als die Aminolyse. Aus diesen Gründen hat sich die katalytisch-enzymatische Aminolyse von FAME in Jet Fuel als bevorzugte Durchführung des Verfahrens durchgesetzt.

**Patentansprüche**

1. Verfahren zur quantitativen Bestimmung von Fettsäureestern in Treibstoffen, **dadurch gekennzeichnet, dass** der Analyt einer für ihn selektiven, die Intensität der Carbonylbande der jeweiligen Estergruppe beeinflussenden chemischen Reaktion unter Bildung eines modifizierten Analyten unterzogen und die Änderung der Konzentration an Analyt in der Probe unter Heranziehung der Abnahme der Intensität der Carbonylbande und/oder die Zunahme der Konzentration des modifizierten Analyten unter Heranziehung der Zunahme der Intensität einer für die Modifizierung charakteristischen Bande gemessen wird.

2. Verfahren nach Anspruch 1, **dadurch gekennzeichnet, dass** die Messung der Änderung der Konzentration an Analyt durch IR-Spektroskopie erfolgt.

3. Verfahren nach Anspruch 2, **dadurch gekennzeichnet, dass** die Messung der Änderung der Konzentration an Analyt durch FTIR-Spektroskopie erfolgt.

4. Verfahren nach Anspruch 2, **dadurch gekennzeichnet, dass** die Messung der Änderung der Konzentration an Analyt durch IR-Spektroskopie unter Verwendung von Quantenkaskadenlaser erfolgt.

5. Verfahren nach einem der Ansprüche 1 bis 4, **dadurch gekennzeichnet, dass** FAME/FAEE/Glycerid durch Zugabe

eines Amins in das entsprechende Amid umgewandelt wird, wobei die Änderung der Konzentration an FAME/FAEE/Glycerid bzw. allgemein die Änderung der Konzentration an zu bestimmenden Ester bzw. Fettsäureester in der Probe anhand der Abnahme der Intensität der Carbonylbande des Esters oder/oder der Zunahme der Intensität der Amidbande des gebildeten Amids gemessen wird.

6. Verfahren nach einem der Ansprüche 1 bis 4, **dadurch gekennzeichnet, dass** der Ester bzw. FAME/FAEE/Glycerid durch Zugabe eines anderen Alkohols unter geeigneten Reaktionsbedingungen in den entsprechenden anderen Ester umgewandelt und die Änderung der Konzentration an FAME/FAEE/Glycerid bzw. allgemein die Änderung der Konzentration an zu bestimmenden Ester bzw. Fettsäureester in der Probe anhand der Abnahme der Intensität der Carbonylbande des Esters und/oder der Zunahme der Intensität der Carbonylbande des anderen Esters gemessen wird.

7. Verfahren nach Anspruch 5 oder 6, **dadurch gekennzeichnet, dass** die Reaktion enzymatisch katalysiert ist.

8. Verfahren nach einem der Ansprüche 2 bis 7, **dadurch gekennzeichnet, dass** die Messung der Änderung der Konzentration an Analyt durch IR-Spektroskopie anhand der Abnahme der Carbonylbande bei 1749 $cm^{-1}$ bzw. 1742 $cm^{-1}$ erfolgt.

9. Verfahren nach Anspruch 8, **dadurch gekennzeichnet, dass** die Messung der Änderung der Konzentration an Analyt durch IR-Spektroskopie anhand der Abnahme der Carbonylbande bei 1749 $cm^{-1}$ durch symmetrische Auswertung um 1742 $cm^{-1}$ erfolgt bzw. anhand der Abnahme der Carbonylbande bei 1742 $cm^{-1}$ durch symmetrische Auswertung um 1749 $cm^{-1}$ erfolgt.

Fig. 1:

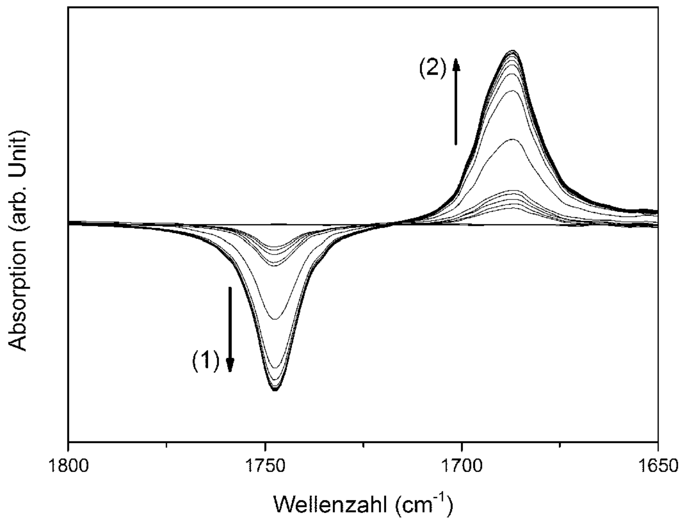
(2)
(1)
Absorption (arb. Unit)
1800
1750
1700
1650
Wellenzahl (cm-1)

Fig. 2:

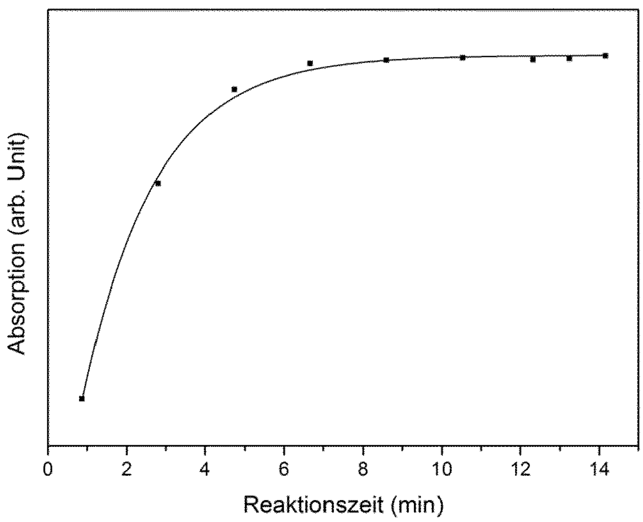
Absorption (arb. Unit)
0
2
4
6
8
10
12
14
Reaktionszeit (min)

Fig. 3:

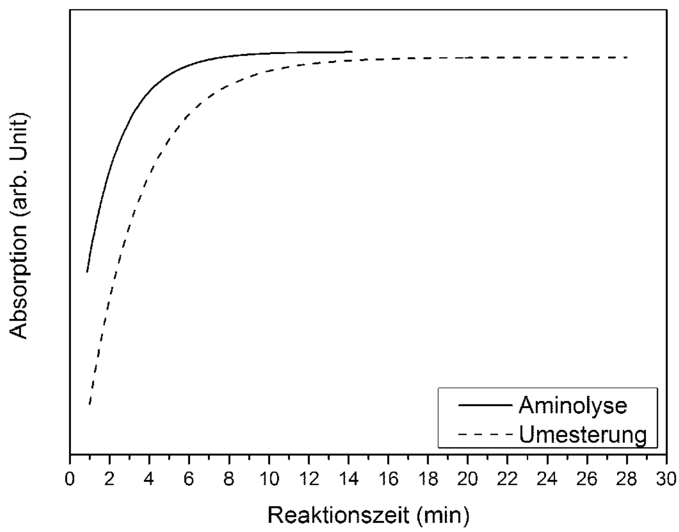
Absorption (arb. Unit)
Aminolyse
Umesterung
0 2 4 6 8 10 12 14 16 18 20 22 24 26 28 30
Reaktionszeit (min)

Europäisches Patentamt
European Patent Office
Office européen des brevets

# EUROPÄISCHER RECHERCHENBERICHT

Nummer der Anmeldung
EP 17 18 9615

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X<br><br><br><br><br><br><br><br><br><br><br><br>Y | MUHAMMAD TARIQ ET AL: "Activity of homogeneous and heterogeneous catalysts, spectroscopic and chromatographic characterization of biodiesel: A review", RENEWABLE AND SUSTAINABLE ENERGY REVIEWS., Bd. 16, Nr. 8, 30. August 2012 (2012-08-30), Seiten 6303-6316, XP055446618, US ISSN: 1364-0321, DOI: 10.1016/j.rser.2012.07.005<br>* Absatz [4.2.2.2] *<br>----- | 1,2,5-9<br><br><br><br><br><br><br><br><br><br><br><br>2-4 | INV.<br>G01N21/3577<br>G01N33/28<br>G01N21/35 |
| Y | Gerhard Knothe: "Monitoring a Progressing Transesterification Reaction by Fiber-Optic Near Infrared Spectroscopy with Correlation to 1H Nuclear Magnetic Resonance Spectroscopy", , 2000, XP055446573, Gefunden im Internet: URL:http://lib3.dss.go.th/fulltext/Journal/J.AOCS/J.AOCS/2000/no.5/may2000vol77,no5,p489-493.pdf [gefunden am 2018-01-31]<br>* Seite 489, rechte Spalte, Absatz 2 - Seite 492, rechte Spalte, letzter Zeile; Abbildungen 1,2 *<br>-----<br>-/-- | 2 | RECHERCHIERTE SACHGEBIETE (IPC)<br>G01N |

Der vorliegende Recherchenbericht wurde für alle Patentansprüche erstellt

1

| Recherchenort | Abschlußdatum der Recherche | Prüfer |
|---|---|---|
| München | 31. Januar 2018 | Consalvo, Daniela |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
&: Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 17 18 9615

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| Y | ALCARÁZ MIRTA R ET AL: "EC-QCL mid-IR transmission spectroscopy for monitoring dynamic changes of protein secondary structure in aqueous solution on the example of beta-aggregation in alcohol-denaturated alpha-chymotrypsin", ANALYTICAL AND BIOANALYTICAL CHEMISTRY, SPRINGER, DE, Bd. 408, Nr. 15, 23. März 2016 (2016-03-23), Seiten 3933-3941, XP035867778, ISSN: 1618-2642, DOI: 10.1007/S00216-016-9464-5 [gefunden am 2016-03-23] * Zusammenfassung; Abbildung 1 * ----- | 3,4 | |
| | | | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 31. Januar 2018 | Consalvo, Daniela |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........
& : Mitglied der gleichen Patentfamilie, übereinstimmendes Dokument

EPO FORM 1503 03.82 (P04C03)